# EUROPEAN PATENT SPECIFICATION

(11) **EP 2 401 253 B1**
(45) Date of publication and mention of the grant of the patent: **13.01.2016**
(21) Application number: 10719784.0
(22) Date of filing: 25.01.2010
(51) Int. Cl.: C07D 207/20, C07D 211/70, C07D 213/61

(54) **A PROCESS FOR THE PREPARATION OF ETORICOXIB**
VERFAHREN ZUR HERSTELLUNG VON ETORICOXIB
PROCÉDÉ DE PRÉPARATION D'ÉTORICOXIB

(30) Priority: 27.02.2009 IN MU04412009
(43) Date of publication of application: 04.01.2012
(60) Divisional application: 12162558.6
(73) Proprietor: Cadila Healthcare Limited, Ahmedabad 380 015 Gujarat (IN)
(72) Inventor: PANDEY, Bipin, Ahmedabad 380 015 Gujarat (IN); DAVE, Mayank, Ghanshyambhai, Ahmedabad 380 015 Gujarat (IN); SHAH, Mitesh, Ahmedabad 380 015 Gujarat (IN)
(74) Representative: Srinivasan, Ravi Chandran
(86) International application number: PCT/IN2010/000045
(87) International publication number: WO 2010/097802

(56) References cited:
- JP-A- 2003 160 563
- DAVIES I W ET AL: "A PRACTICAL SYNTHESIS OF A COX-2-SPECIFIC INHIBITOR" JOURNAL OF ORGANIC CHEMISTRY, AMERICAN CHEMICAL SOCIETY, EASTON.; US LNKD- DOI:10.1021/JO000870Z, vol. 65, 1 January 2000 (2000-01-01), pages 8415-8420, XP002326845 ISSN: 0022-3263
- DAVIES, IAN W. ET AL: "Preparation and Novel Reduction Reactions of Vinamidinium Salts" JOURNAL OF ORGANIC CHEMISTRY , 66(1), 251-255 CODEN: JOCEAH; ISSN: 0022-3263, 2001, XP002592388

## Description

### FIELD OF INVENTION

The present invention relates to an improved process for the preparation of Etoricoxib and also a process for preparing substituted β-chlorovinamidinium salts with cyclic alkyl group (with or without hetero atom), which is one of the intermediate for preparation of Etoricoxib.

### BACKGROUND OF THE INVENTION

Etoricoxib is a selective COX-2 inhibitor which has been shown to be as effective as non-selective non-steroidal anti-inflammatory drugs in the management of chronic pain in rheumatoid arthritis, osteoarthritis and other COX-2 mediated disorders. Etoricoxib is 5-chloro-6'-methyl-3-[4-methylsulfonyl)phenyl]-2,3'-bipyridine having structural formula (I).

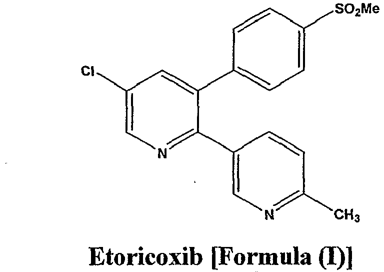

Etoricoxib belongs to a class of drugs known as COX-2 inhibitors that are used in the treatment of COX-2 mediated disorders. The therapeutic application of Etoricoxib as a COX-2 inhibitor is disclosed in WO 9610012 and WO 9616934.

Compounds of general formula (X) which includes Etoricoxib are disclosed in WO9803484 (US 5861419). Process for the preparation of Compound of formula (X) involves bromination of 2-amino pyridine derivative with bromine in acetic acid to provide the bromide of 2-amino pyridine derivative. Coupling of this bromide derivative with 4-(methylthio)phenyl-boronic acid is done in presence of a suitable base, which is then oxidized to give the corresponding sulphon. Then the amino group of sulphon is converted to corresponding halide. A second palladium catalyzed coupling of sulphon halide derivative with an appropriately substituted metal containing aromatic give compound of formula (X).

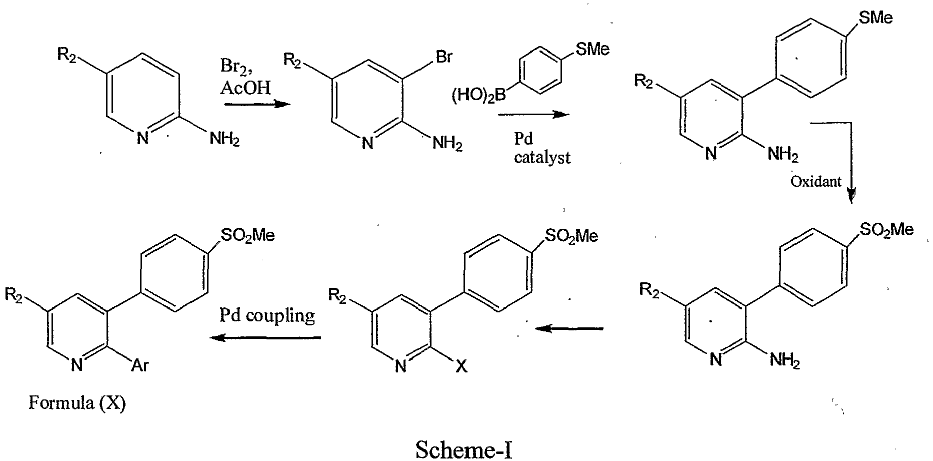

WO9803484 application also discloses a second method for the preparation of compound of formula (X), which is as per scheme II.

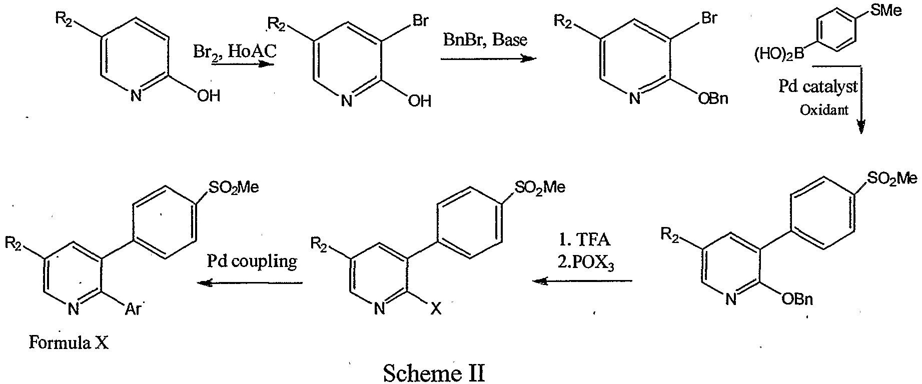

US 6812346 discloses a method for the preparation of Etoricoxib which comprises reacting a compound of formula B, wherein X is Br or Cl, with compound of formula C, wherein M is B(OH)₂ or SnMe₃, in the presence of a palladium catalyst to yield Etoricoxib.

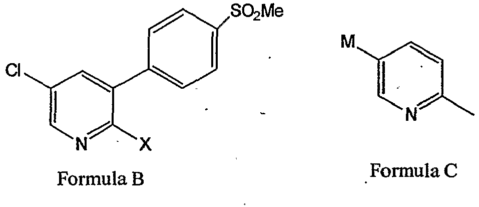

A process for the preparation of Etoricoxib as described in scheme III, is disclosed in US6040319, wherein X represents phosphates, sulfates, acetates, perchlorate, borates, antimonates, halides, benzoate, napsylate, particularly, hexafluorophosphate, sulfate, mesylate, tosylate, triflate, acetate, trifluoroacetate, tetrafluoroborate, tetraphenyl borate, hexafluoroantimonate, chloride, bromide, fluoride, iodide, benzolate and napsylate. Base used is Na and K hydroxide; Cs carbonate; Li, Na and K C₁₋₆ alkoxide; Li, Na and K amides, Li, Na and K hydrides.

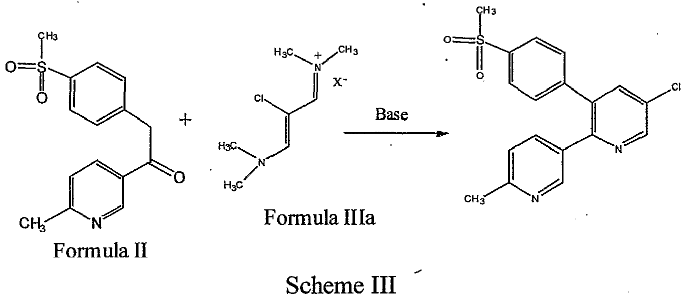

WO 0137833 discloses different polymorphs of Etoricoxib, specifically disclosed are forms II, III and IV, hemihydrate of form IV and sesquihydrate of form I.
WO 0192230 discloses polymorphic form V of Etoricoxib and process for its preparation. WO 0296877 discloses pharmaceutical composition comprising 10-50% of polymorphic form V and remainder of the compound comprising at least one polymorph selected from forms I, II, III and IV.

WO 2005085199 discloses different other polymorph of Etoricoxib, specifically disclosed forms IX, X, XI, XII, XIII, XIV, XV and XVI.
US 6252116 (divisional of US 6040319) discloses process for preparing intermediate of formula (III), particularly CDTH (2-chloro-1,3-bis(dimethylamino)trimethinium hexafluorophosphate) salt with hexafluorophosphate, tetraphenyl borate, hexafluoroantimonate as it counterion (X⁻).

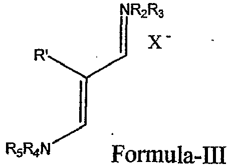

According to formula (III) R₂ through R₅ each independently represents C₁₋₆ alkyl, aryl or aralkyl group.

Certain heteroatom containing cycloalkyl substituted β-chlorovinamidinium salts are disclosed in J. Org. Chem., Vol. 66, No. 1, p. 251-255, 2001. But a large number of limitations are also reported. The method disclosed for the preparation of substituted β-chlorovinamidinium salt comprises, addition of chloroacetic acid to N-formyl compound containing a cycloalkyl group which optionally contains a hetero atom and the mixture was heated at 70 °C and then to it was added phosphorus oxychloride which after suitable work up gave the substituted β-chlorovinamidinium salt. This document also states that the β-chlorovinamidinium salt using N-formylmorpholine was obtained with very low yield (<10%) and pure sample could not be obtained by using this method. Thus, there exists a need to prepare suitable substituted β-chlorovinamidinium salts containing a cyclic group optionally containing one or more heteroatom, as one of the intermediate for the preparation of Etoricoxib, in a pure form and high yield, and preparing

Etoricoxib using this intermediate, in a cost effective and industrially scalable way. We herein disclose an improved process for preparation of differently substituted β-chlorovinamidinium salts containing a cyclic group optionally containing one or more heteroatom, and also a process for the preparation Etoricoxib using the substituted β-chlorovinamidinium salts thus obtained.

### BRIEF DESCRIPTION OF THE DRAWINGS

FIG. 1 is a powder X-ray diffraction (XRPD) pattern of the Etoricoxib obtained according to the process of the present invention.
FIG. 2 is a powder X-ray diffraction (XRPD) pattern of form I of 2-chloro 1,3-(bis piperidyl) trimethinium hexafluoro phosphate according to the present invention.
FIG. 3 is a powder X-ray diffraction (XRPD) pattern of form II of 2-chloro 1,3-(bis piperidyl) trimethinium hexafluoro phosphate according to the present invention.

### OBJECTS OF THE INVENTION

The main objective of the present invention is to provide improved process for preparing Etoricoxib.

In an embodiment is provided an improved process for preparing substituted β-chlorovinamidinium salts of formula (IIIb) 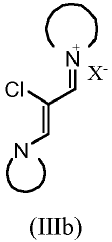 wherein 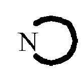 represents a cyclic group, optionally containing one or more heteroatom.

In a preferred embodiment is provided an improved process for preparing substituted β-chlorovinamidinium salts of formula (IIIb).

In another embodiment is provided a process for preparing Etoricoxib of formula (I) using the intermediate of formula (IIIb).

In one further embodiment is provided a process for the purification of substituted β-chlorovinamidinium salts of formula (IIIb).

In one of the embodiment of the present invention is disclosed a novel intermediate of formula (IIIc) and use of this intermediate in the process of preparation of Etoricoxib. The above and other embodiments are further described in the following paragraphs.

### DETAILED DESCRIPTION

As used herein, the term "THF" refers to tetrahydrofuran, the term "DMF" refers to dimethyl formamide, the term "DIPE" refers to di-isopropyl ether, the term "DMSO" refers to dimethyl sulphoxide, the term "MTBE" refers to methyl t-butyl ether, the term, "MEK" refers to methyl ethyl ketone, the term 'LHMDS' refers to lithium bis (trimethylsilyl)amide, the term 'KHMDS' refers to potassium bis (trimethylsilyl)amide.

The present invention provides, an improved process for the preparation of Etoricoxib which involves reacting substituted β-chlorovinamidinium salt of formula (IIIb) and a ketosulfone compound of formula (II). 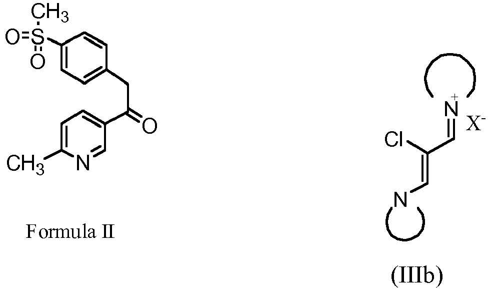 wherein 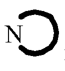 represents a cyclic group, optionally containing one or more heteroatom selected from N, O or S & 'X⁻' represents the suitable counterion.

Thus, the present invention provides a process for the preparation of Etoricoxib of formula-(I) comprising, 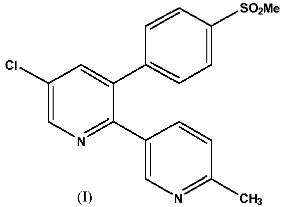
(a) reacting a compound of formula-(IV) with suitable chloroacetyl halide or chloro acetic acid and phosphorous oxychloride to obtain the compound of formula (IIId) & further converting the compound of formula (IIId) into its suitable salt to obtain the compound of formula-(IIIb), 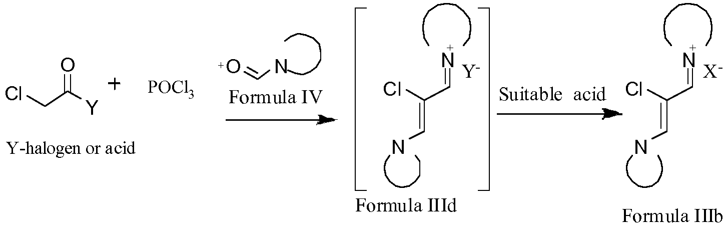 wherein 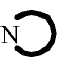 represents a cyclic group, optionally containing one or more heteroatoms selected from N, O or S & 'X⁻' represents a suitable counter ion.
(b) reacting the compound of formula (IIIb) with compound of formula (II) in presence of suitable base and suitable solvent to obtain Etoricoxib of formula (I)

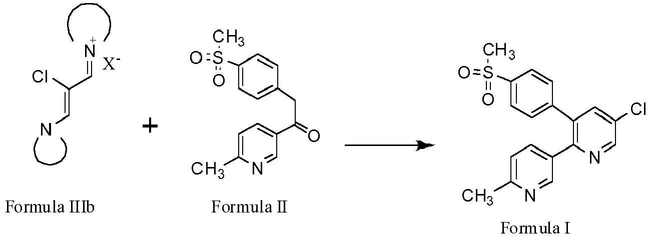

Preferably, the groups comprising 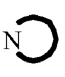 may be selected from morpholidinyl, piperidinyl and a pyrolidinyl group.

Preferably, the suitable salt of compound of formula (IIIb) is prepared by reaction of the compound of formula (IIId) with a suitable salt of an acid to obtain corresponding salt of compound formula (IIIb).

Preferably, the suitable salt of an acid is selected from the group consisting of phosphate, sulphate, acetates, perchlorate, borates, antimonite, halides, benzoate, napsylate, hexafluorophosphate, tetrafluoro borate, chloride, bromide, fluoride, iodide, benzoate and carbonate.

Preferably, the suitable salt of acid is hexafluorophosphate.

Preferably, the suitable solvent is selected from C₁-C₆ alcohols, hydrocarbons selected from benzene, toluene and xylene; halocarbon solvents selected from mono or di halo C₁-C₄ alkyls, nitrogen containing solvents selected from DMF, dimethyl acetamide, N-ethylpyrrolidinone, N-methyl pyrrolidinone and acetonitrile and dioxane and dimethyl sulfoxide.

Preferably, the solvents are selected from C₁-C₆ alcohol, THF, DMF, dioxane and dimethyl sulfoxide.

Preferably, the suitable base used is selected from suitable organic or inorganic bases, wherein the inorganic bases are selected from alkali metal or alkaline earth metal hydroxides, alkali metal or alkaline earth metal carbonates such as NaHCO₃, Na₂CO₃, K₂CO₃, alkali metal hydrides, alkali metal alkoxides.

The present invention further provides a process for the preparation of β-chlorovinamidinium salt of formula (IIIb).

The present invention also includes a new intermediate of formula (IIIc) for the preparation of Etoricoxib.

The present invention provides a process for the preparation of substituted β-chlorovinamidinium salt of formula (IIIb) comprising, reacting a compound of formula (IV), wherein 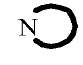 is as defined earlier, with a suitable chloroacetyl halide or chloro acetic acid and phosphorous oxychloride at room temperature or below, and adding the compound of formula (IV) to the combination to obtain the compound of formula (IIId) & further converting the compound of formula (IIId) into its suitable salt of formula-(IIIb).

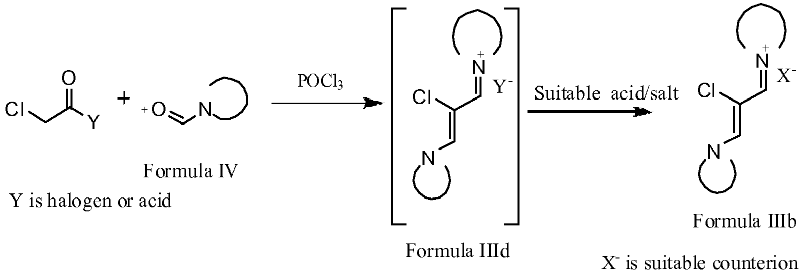

Preferably, the temperature is less than 30 °C.

Preferably, the process comprises combining chloro acetic acid or a sutable chloroacetyl halide and phosphorous oxychloride, and thereafter adding the compound of formula (IV).

Preferably, the N-formyl compound of formula (IV) is added drop wise to the combination of the chloro aceticacid or chloroacetyl halide and phosphorous oxychloride. Preferably, prior to the addition of N-formyl compound, a cooling step is performed. Preferably, the cooling is to a temperature of -10 °C to 25 °C. More preferably cooling is done to 10 °C to 20 °C and most preferably 0 °C to 10 °C.

Preferably, after combining chloro acetic acid or suitable chloroacetyl halide, phosphorous oxychloride and N-formyl compound of formula (IV), as above, the reaction mixture was heated to a suitable temperature for suitable period of time.

Preferably, the suitable temperature during this step may be selected from 50 °C to 150 °C, more preferably from 75 °C to 120 °C and most preferably from 90 °C to 100 °C.

Preferably, the reaction mixture is maintained at the desired high temperature for 3 hour to 25 hours, more preferably for 5 to 10 and most preferably for 6 to 8 hrs.

### Recovery of the β-chlorovinamidinium salt:

The reaction mixture was cooled to room temperature to obtain a semi solid mass of compound of formula (IIId), which was dissolved in a suitable organic solvent and subsequently added to a suitable acid or a salt of suitable acid at suitable temperature over a suitable period of time. The pH of the reaction mixture was maintained at pH 3-5 using aqueous solution of a suitable base. The β-chlorovinamidinium salt of formula (IIIb) was isolated by adding water. Preferably, the isolation is done by filtration.

Preferably, the organic solvent used for obtaining the salt may be selected from the group comprising of dimethylformamide (DMF), alcohols like methanol, ethanol, isopropanol, butanol, 1,2-dimethoxy ethanol, 2-methoxy ethanol, 2-ethoxy ethanol, ethylene glycol and the like and their suitable mixtures with water.

Preferably, the temperature at this step is maintained at 0 °C to 25 °C, more preferably 10 °C to 20 °C and most preferably at 0 °C to 10 °C.

Preferably, the compound of formula (IIId) is converted to its suitable salt of formula (IIIb) by reacting it with the salt of corresponding acids.

Preferably, salt of suitable acids may be selected from the group comprising of hexafluorophosphate, phosphate, sulphate, acetates, perchlorate, borates, antimonite, halides, benzoate, napsylate, tetrafluoro borate, chloride, bromide, fluoride, iodide, benzoate, carbonate, hexafluoroantimonate.

In an embodiment the invention encompasses process for purification of the β-chlorovinamidinium salt of formula (IIIb).

The process of purification comprises dissolving the β-chlorovinamidinium salt of formula (IIIb) in a mixture of suitable solvent(s) and water and stirring the reaction mixture. Subsequently the reaction mixture was washed with suitable solvent(s) and water to obtain the β-chlorovinamidinium salt with > 95 % purity.

The suitable solvent used in the purification step may be selected from C₁-C₅ alcohols or their suitable esters or suitable mixtures thereof.

The β-chlorovinamidinium salt of formula (IIIb) obtained above was further purified (having from 1 to 3 % total impurity) by taking the β-chlorovinamidinium salt of formula (IIIb) in a suitable solvent and adding aqueous solution of a bisulphite or metabisulphite & subsequently washing the solution with water. Suitable solvent for the second purification may be selected from C₁-C₅ alcohols and suitable esters or suitable mixtures thereof. Alcohols are preferred.

The present invention also provides a method of producing a compound of formula-(IIIb) as above, wherein the compound of formula (IIIb) is further purified by
i) dissolving the compound of formula (IIIb) in C₁-C₅ alcohols or their esters, optionally with water & subsequently isolating the compound of formula (IIIb);
ii) treating the compound of formula (IIIb) with suitable alkali bisulphite or metabisulphite, in C₁-C₅ alcohols or their esters and subsequently isolating the pure compound of formula (IIIb).

The preferred bisulphite or metabisulphite used for the second purification may be selected from sodium or potassium bisulphite or metabisulphite.

Preferably, the salt of formula (IIIb) is obtained with at least 99 % purity by HPLC.

The present invention also provides a compound of structural formula-(IIIc), wherein 'X⁻' represents a suitable counter ion.

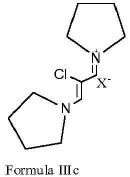

In one of the embodiment of the invention is disclosed 2-chloro 1,3-(bis piperidyl) trimethinium hexafluoro phosphate and 2-chloro 1,3-(bis pyrolidinyl) trimethinium hexafluoro phosphate salt with at least 99.9 % purity by HPLC.

It has surprisingly been found according to the invention that 2-chloro 1,3-(bis piperidyl) trimethinium hexafluorophosphate is obtained in two different polymorphic forms, which are designated as Form-I & Form-II respectively.

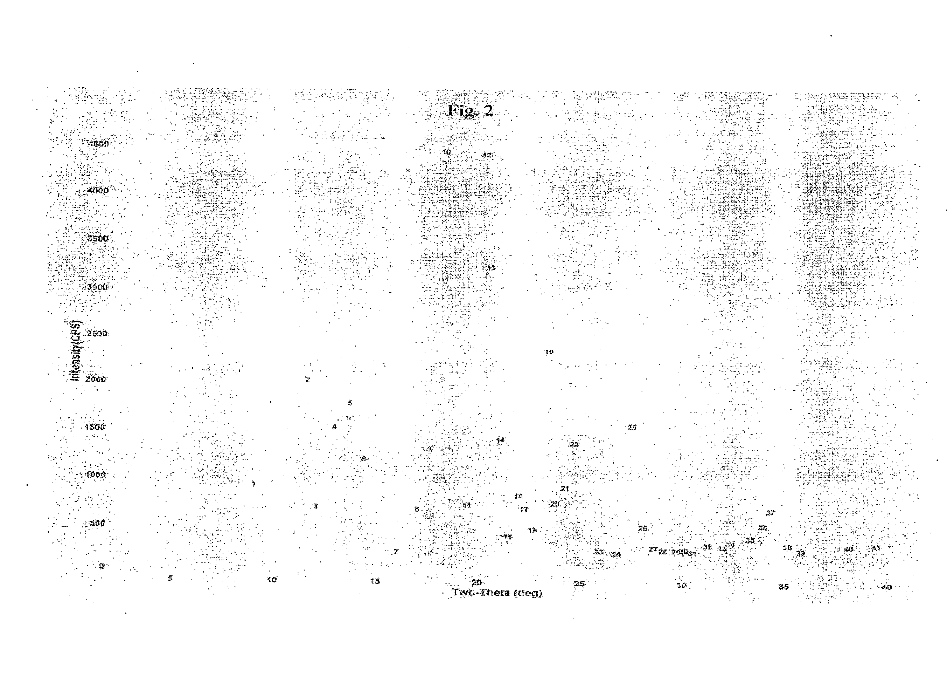

Form I of 2-chloro 1,3-(bis piperidinyl) trimethinium hexafluorophosphate obtained according to the present invention is characterized by an XPRD pattern substantially in accordance with the pattern disclosed in Fig. 2.

Form I of 2-chloro 1,3-(bis piperidinyl) trimethinium hexafluoro phosphate obtained according to the present invention is also characterized by an XPRD peaks at about 9.06, 11.60, 12.06, 12.89, 13.64, 14.38, 16.022, 16.98, 17.56, 18.16, 19.43, 20.16, 20.46, 21.04, 21.48, 21.98, 22.25, 22.68, 23.35, 23.76, 24.24, 24.64, 25.92, 26.75, 27.42, 28.03, 28.55, 29.04, 29.68, 30.06, 30.52, 31.25, 31.96, 32.36, 33.31, 33.94, 34.30, 35.16, 35.80, 38.14, 39.47 ° ± 0.2 ° degrees (2θ)( Fig. 2 ) and has melting point in the range of 185-200 °C. (Decomposed)

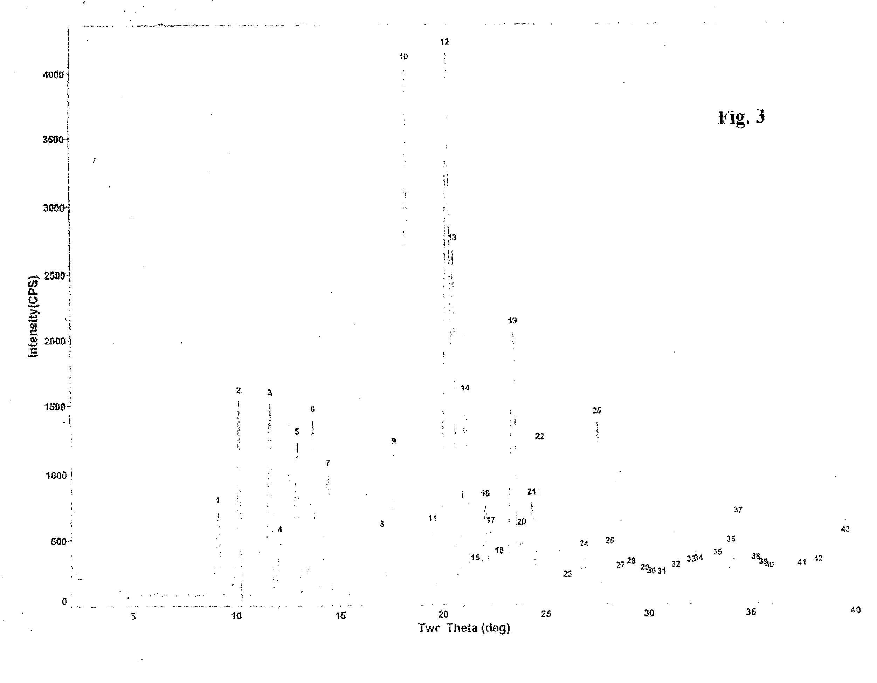

Form II of 2-chloro 1,3-(bis piperidinyl) trimethinium hexafluoro phosphate obtained according to the present invention is also characterized by an XPRD peaks at about 9.14, 10.142, 12.12, 12.96, 13.71, 17.06, 17.64, 18.24, 19.49, 20.520, 21.10, 21.56, 22.32, 22.74, 23.42, 24.32, 24.72, 26.05, 27.48, 28.10, 29.14, 29.79, 30.12, 30.61, 31.30, 32.06, 32.43, 33.36, 34.02, 34.38° ± 0.2 ° degrees (2θ) (Fig. 3) and has melting point in the range of 190-197 °C.

The present invention further provides a process for the preparation of Etoricoxib, using the β-chlorovinamidinium salt of formula (IIIb), which comprises of the following steps:
(a) reacting a compound of formula-(IV) with suitable chloroacetyl halide or chloro acetic acid and phosphorous oxychloride to obtain the compound of formula (IIId) & further converting the compound of formula (IIId) into its suitable salt to obtain the compound of formula-(IIIb), as described above; and
(b) reacting compound of formula (IIIb) with compound of formula (II) in presence of suitable base and suitable solvent to obtain compound of formula (I).

The above process may be illustrated as per the following Scheme IV:

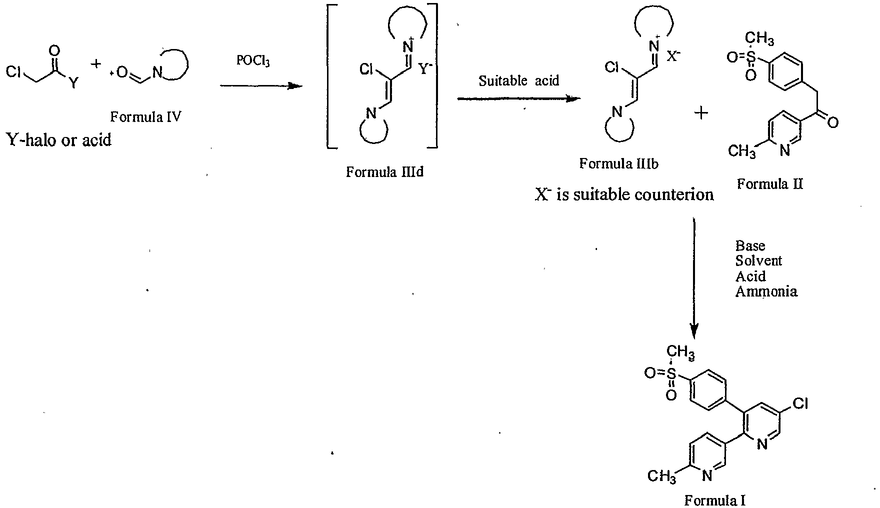

Preferably, the groups comprising 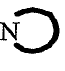 may be selected from morpholidinyl, piperidinyl and pyrolidinyl group.

Preferably, step (a) further comprises the preparation of compound of formula (IIIb) as described above.

Preferably, the β-chlorovinamidinium salt of formula (IIIb) is as described above. Preferably, step (a) comprises first combining chloroacetyl chloride and phosphorous oxychloride, and thereafter combining the N-formyl compound of formula (IV), to obtain the β-chlorovinamidinium salt of formula (IIIb), by processes as described above. Preferably, the temperature in step (a) is as described above.

Preferably, the reaction mixture is maintained in step (a) is as described above. Prior to step (b), the β-chlorovinamidinium salt of formula (IIIb) obtained in step (a) is separated. Preferably, the separation is done by filtration as described earlier in the specification.

The organic solvent in step (b) may be selected from the group comprising of suitable hydrocarbons such as benzene, toluene, xylene, ethyl benzene, trimethyl benzene & the like; suitable halogenated hydrocarbon solvents such as chloroform, dichloromethane, dichloroethane and the like or mixtures thereof; alcohols such as methanol, ethanol, t-butanol and like or mixtures thereof; ketones such as acetone; aprotic solvents such as DMF, dimethyl acetamide; ethers like diethyl ether, 1,4-dioxane, DIPE, MTBE, THF & the like, aprotic polar solvents such as DMF, DMSO, DMA; nitriles like acetonitriles or their suitable mixtures thereof.

The suitable base used in step (b) may be selected from suitable organic or inorganic bases such as hydroxides selected from NaOH, KOH & the like, carbonates such as NaHCO₃, Na₂CO₃, K₂CO₃, hydrides such as NaH and LHMDS, KHMDS and like; alkali metal alkoxides such as sodium, potassium, lithium t-butoxide and like; sodium, potassium, lithium isopropoxide and like.

Optionally, after completion of step (b) compound formula (I) can be isolated or subjected to insitu purification. Purification of compound formula (I) may be done by using formalin solution and aq. ammonia in suitable solvent. The reaction mixture is stirred at suitable temperature and product is isolated using suitable solvent. Then after recrystallization is carried out using suitable solvent to obtain pure Etoricoxib.

The suitable solvent used for the purification of Etoricoxib may be selected from C₁-C₅ alcohol; ketones such as acetone and methyl ethyl ketone; ethers such as THF and dioxane or their suitable mixtures.

The suitable solvent used for the isolation of Etoricoxib may be selected from suitable alcohols or esters. The preferred solvent is isopropyl alcohol.

The suitable solvent used for recrystallization of Etoricoxib may be selected from suitable alcohols, esters, ethers or their suitable mixtures. The preferred solvent for recrystallisation is isopropyl alcohol.

In one of the embodiment of the invention is disclosed Etoricoxib obtained according to the process of the present invention with at least 99.9 % purity by HPLC.

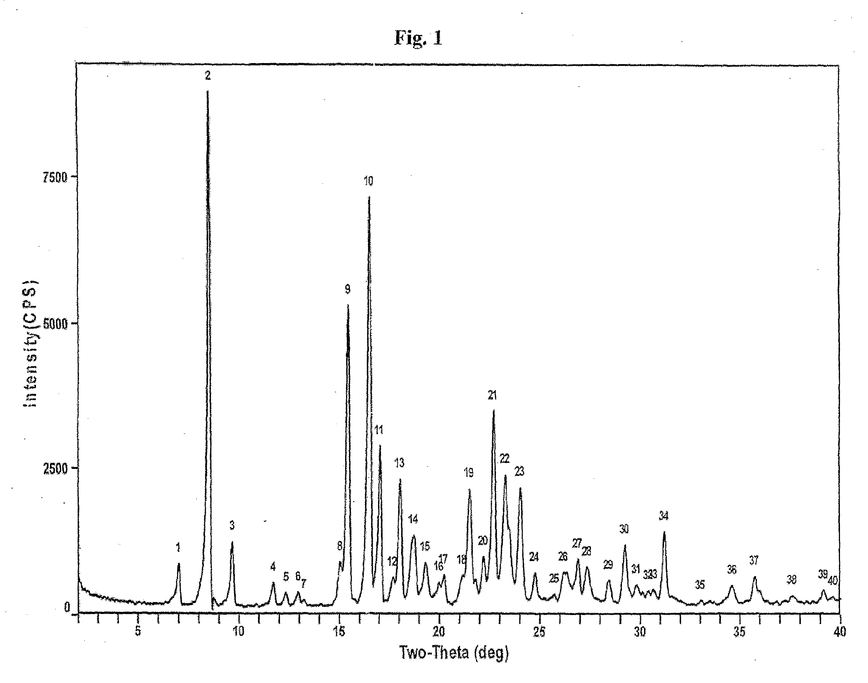

Etoricoxib obtained according to the process of the present invention is characterized by an XPRD pattern substantially in accordance with the pattern Fig. 1.

Etoricoxib obtained according to the present invention is also characterized by an XPRD peaks at 7.00, 8.50, 9.67, 11.72, 12.35, 12.96, 13.24, 15.04, 15.44, 16.51, 17.06, 17.70, 18.07, 18.76, 19.34, 20.00, 20.24, 21.14, 21.50, 22.20, 22.70, 23.28, 24.02, 24.76, 25.73, 26.20, 26.90, 27.37, 28.44, 29.24, 29.83, 30.41, 30.66, 31.22, 33.04, 34.63, 35.76, 37.62, 39.16, 39.65 ° ± 0.2 ° degrees (2θ) (Fig.1) and has melting point in the range of 135-137 °C.

In one of the embodiment of the present invention is disclosed a novel intermediate of formula (IIIc), and use of this intermediate in the process of preparation of Etoricoxib.

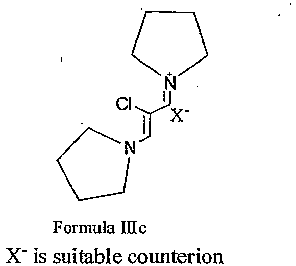

The 2-chloro 1,3-(bis pyrolidinyl) trimethinium hexafluoro phosphate salt was isolated and characterized by the following data:
**¹H NMR** (300MHz, DMSO-D6) δ (ppm): 1.82 - 1.95 (8 H, dd), 3.73 (4H, s), 4.01 (4H, s), 7.98 (2H, s);
**¹³C NMR** (75MHz, DMSO-D6) δ (ppm): 23.14, 25.50, 49.51, 56.73, 92.70, 156.53
**ESI MS** = 212.9 m/z (base peak).

In a further embodiment of the invention is disclosed a pharmaceutical composition of Etoricoxib with a liquid or solid carrier, excipients as is known in the art wherein the Etoricoxib is produced by the above disclosed process.

The invention is further described by the following examples, which are provided for illustration only and should not be construed to limit the scope of invention.

### Example 1

### Preparation of 2-chloro 1,3-(bis morpholinyl) trimethinium hexafluoro phosphate

Phosphorous oxychloride (6.6g) was added in to N-formyl morpholine (5g) at -5 to 10 °C and subsequently chloroacetyl chloride (5g) was added at room temperature and the reaction mixture was stirred. Further, N-formyl morpholine (5g) was added to the reaction mixture at room temperature and the reaction mass was heated at 55-110 °C for 3 to 24 hrs. The reaction mixture was then cooled to room temperature to obtain a semi solid mass, which was dissolved in dichloromethane and subsequently a solution of NaPF₆ was added at 0-10 °C over a period of one hour. Then into it 50 ml water was added, organic layer was separated, dried and concentrated till dry at less than 50 °C, to give 2-chloro 1,3-(bis morpholinyl) trimethinium hexafluoro phosphate. Yield=(4 g). HPLC purity = 44.63 %.

### Example 2

### Preparation of Etoricoxib

Potassium tert. butoxide (1.4g) was dissolved in THF and the solution was maintained at 5-25 °C for 10 minutes. Subsequently, a solution containing the 1-(6-methylpyridin-3-yl)-2[4-(methylsulfonyl)phenyl] ethanone (3g) in THF was added and kept this at 25-30 °C for 1 hr. 2-chloro 1,3-(bis morpholinyl) trimethinium hexafluoro phosphate of (formula III) (4g) in THF, obtained above, was added to the previously prepared solution of 1-(6-methylpyridin-3-yl)-2[4-(methylsulfonyl)phenyl] ethanone and the reaction mixture was heated at 30-55 °C for 2-24 hrs. The reaction mixture was dumped into a mixture of trifluoroacetic acid (0.6 g), acetic acid (4.4g) and THF and further heated at 55-60 °C for 6-20 hrs after adding aqueous ammonia solution (30 ml) and ammonium acetate (0.8g). The reaction mixture was dumped into water and extracted with ethyl acetate and washed with 10% aqueous sodium bicarbonate solution. The organic layer was separated, dried & concentrated till dry at temperature less than 50 °C to obtain Etoricoxib. Yield = (3.1 g),
% purity by HPLC = 15.36 % (Etoricoxib), 64.15 % (unreacted ketosulfone compound of formula II

### Example 3 (Reference Example

### Preparation of 2-chloro 1,3-(bis piperidinyl) trimethinium hexafluorophosphate

Chloroacetic acid (4.75g, 0.05 mole) was added to N-formyl piperidine (13.5g, 0.119 mole) and the reaction mixture was heated to 70-75 °C to give a clear solution. Subsequently, phosphorous oxychloride (9.5 ml, 0.1 mole) was added over a period of 1-2 hrs and maintained at 70-75 °C for 3 hrs. The reaction mixture was cooled to room temperature and diluted with absolute alcohol (10 ml) and this reaction mass was added into the solution of NaPF₆. The reaction mass was stirred for 30 minutes at 0-10 °C, filtered and washed with water, to obtain 2-chloro 1,3-(bis piperidinyl) trimethinium hexafluoro phosphate (2 g), yield = 10.42 %, HPLC purity- 83.63 %

### Example 4

### Preparation of 2-chloro 1,3-(bis piperidinyl) trimethinium hexafluoro phosphate

Chloroacetyl chloride (20g) solution was cooled and phosphorous oxychloride (27.2g) was added into it at -10 to 0 °C. Subsequently, N-formyl piperidine (40g) was added over a period of 1 hrs, maintained the reaction mass at 55-110 °C for 6-20 hrs. Then the reaction mixture was cooled to room temperature and diluted with DMF (40 ml). This reaction mass was added into the solution of NaPF₆ over a period of 1 hr and the reaction mixture was stirred for 60 minute at 0-10 °C. The reaction mixture was filtered and washed with water to obtain crude 2-chloro 1,3-(bis piperidinyl) trimethinium hexafluoro phosphate (37.2 g), HPLC purity = 83.24 %.

Crude mass was purified by using methanol: water treatment to obtain 2-chloro 1,3-(bis piperidyl) trimethinium hexafluoro phosphate (18.8 g), yield = 27.48 %, HPLC purity = 99.19 %.

### Example-5

### Preparation of Etoricoxib

Potassium tert. butoxide (2.1g) was dissolved in THF (20ml) and the solution was maintained at 5-25 °C for 10 minutes. Subsequently, a solution of 1-(6-methylpyridin-3-yl)-2[4-(methylsulfonyl)phenyl] ethanone (5g) in THF was added and kept the mixture for 1hr at 25-30 °C. 2-chloro 1,3-(bis piperidyl) trimethinium hexafluoro phosphate salt of formula (IIIc) (8g), obtained in Example 4 above, in THF, was added to the previously prepared solution of ketosulfone and the reaction mixture was heated at 30-55 °C for 2-24 hrs. The reaction mixture was dumped into a mixture of trifloroacetic acid (1g), acetic acid (7.5g) and THF. The reaction mixture was further heated to 55-60 °C for 6-20 hrs, after adding ammonia solution (125 ml). Then cooled the reaction mixture to room temperature. The reaction mixture was dumped into water and extracted with ethyl acetate and washed with 10% aqueous sodium bicarbonate solution. The organic layer was separated and concentrated to dryness at less than 50 °C to give Etoricoxib 8.6 g.
HPLC purity- 91.28 % (Etoricoxib).
Crude mass was purified to obtain Etoricoxib (4.7g), yield = 75.9 %; HPLC purity = 96.31 %.

### Example-6

### Preparation of 2-chloro 1,3-(bis morpholinyl) trimethinium hexafluoro phosphate

Phosphorous oxychloride (6.6 g) was added in to N-formyl morpholine (5g) at a temperature of -5 to 5 °C. Subsequently chloroacetyl chloride (5g) was added at a temperature of 25-30 °C followed by addition of N-formyl morpholine (5g) at temperature 55 to 60 °C, there after the reaction mass was maintained at temperature 80-90 °C for 5 hr, & then cooled to 25-30 °C. The reaction mixture was diluted with dichloromethane 50 ml and the solution of NaPF₆ was added at 0-10 °C over a period of one hour. 50 ml water was added to the reaction mixture and organic layer was separated, dried, and concentrated to dryness at < 50 °C to obtain 2-chloro 1,3-(bis morpholinyl) trimethinium hexafluoro phosphate.
% Yield = 23.13 %, (4 g), HPLC purity = 44.63 %.

### Example-7

### Preparation of Etoricoxib

Potassium tert. butoxide (1.4g) was dissolved in THF (7ml) and the solution was maintained at 25-30 °C for 10 minutes and then cooled to 10 °C. Subsequently, solution of 1-(6-methylpyridin-3-yl)-2[4-(methylsulfonyl)phenyl] ethanone (5g) in THF was added and kept the mixture for 1hr at 25-30 °C. Subsequently, 2-chloro 1,3-(bis morpholinyl) trimethinium hexafluoro phosphate (4g) followed by THF (20ml) was added and the reaction mixture was maintained at 25-30 °C for 2 hrs. The reaction mixture was dumped in to the mixture of trifluoroacetic acid (0.6g), acetic acid (4.4g) and THF (10ml) and maintained the reaction mixture at 25-30 °C for 2 hrs. The reaction mixture was stirred at 55-60 °C for 3 hrs and aq. ammonia solution (30ml), and ammonium acetate (0.8g) was added and further stirred at 55-60 °C for 20 hrs. The reaction mixture was cooled to 25-30 °C then after water was added and extract with ethyl acetate (50ml). Organic layer was separated and washed with 10 % sodium bicarbonate solution, dried and concentrated to dryness < 50 °C to obtain Etoricoxib.
Yield = 3.1 g, (12.82 %), HPLC purity = 15.36.% (Etoricoxib), (unreacted 64.15 %-sulfone compound).

### Example-8

### Preparation of 2-chloro 1,3-(bis pyrolidinyl) trimethinium hexafluoro phosphate salt

Chloroacetyl chloride (5g) was cooled to -10 to 0 °C and phosphorous oxychloride (6.8g) was added in one lot at 0 to 10 °C. Subsequently, N-formyl pyrolidine (8.76g) was added drop wise at 0 to 5 °C and the reaction mixture was maintained at 90-100 °C for 6 hrs. There after the reaction mixture was cooled to 25-30 °C and diluted with DMF (10ml). Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis pyrolidinyl) trimethinium hexafluoro phosphate salt. Yield = 8.7 g, HPLC purity = 93.09 %.
Crude mass was purified using Methanol: water. Yield = 5.2 g, (32.9 %), HPLC purity = 98.87 %.

### Example-9

### Preparation of Etoricoxib

Potassium tert. butoxide (0.426g) was dissolved in THF (40ml) and the solution was maintained at 25-30 °C for 10 minutes and then cooled to 10-15 °C. Subsequently, solution of 1-(6-methylpyridin-3-yl)-2[4-(methylsulfonyl)phenyl] ethanone (1g) in THF was added and kept the mixture for 1hr at 10-15 °C. Subsequently, 2-chloro 1,3-(bis pyrolidinyl) trimethinium hexafluoro phosphate salt (1.3g), in THF (8ml) was added and maintained the reaction mixture for 2 hrs at 25-30 °C. The reaction mixture was dumped in to the mixture of trifloroaceticacid (0.2g), acetic acid (1.5g), and THF (10ml) and maintained for 1 hour at 25-30 °C. Further the reaction mixture was heated at 60-70 °C for 15 hrs after aq. ammonia solution (25ml) was added and the reaction mixture was cooled to 25-30 °C. After then ethyl acetate and water was added, organic layer was separated and washed with water and 10 % sodium bicarbonate solution. Organic layer was separated, dried and concentrated to dryness < 50 °C to give crude Etoricoxib.
Yield = 0.9 g (40.84 %), % HPLC purity = 55.82 % (Etoricoxib), (unreacted sulfone compound = 34.44 %).

### Example-10

### Preparation of 2-chloro 1,3-(bis piperidinyl) trimethinium hexafluoro phosphate

Chloroacetyl chloride (5g) was cooled to -10 to 0 °C and phosphorous oxychloride (6.8g) was added in one lot at 0 to -10 °C. Subsequently, N-formyl piperidine (10g) was added drop wise at 0 to 5 °C and reaction mixture was maintained at 90-100 °C for 16 hrs. Thereafter reaction mixture was cooled to 25-30 °C and diluted with DMF. Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
% Yield = 15.4 g (61.23 %), HPLC purity = 68.84 %.

### Example-11

### Preparation of 2-chloro 1,3-(bis piperidinyl) trimethinium hexafluoro phosphate

Phosphorous oxychloride (6.6g) was added in to N-formyl piperidine (6.1g) at temperature 15 to 17 °C. Subsequently chloroacetyl chloride (4.7g) was added drop wise at temperature 0-10 °C followed by addition of N-formyl piperidine (6.1g) in one lot at temperature 5 to 10 °C, then after the reaction mass was maintained at temperature 70-75 °C for 3 hr, cooled to 25-30 °C. The reaction mixture was diluted with DMF and the solution of NaPF₆ was added at 0-10 °C over a period of one hour. The reaction mixture was stirred for 60 minutes at 0-10 °C, filtered and washed with water, dried to obtain 2-chloro 1,3-(bis piperidinyl) trimethinium hexafluoro phosphate.
Yield = 3 g, (18.75 %), HPLC purity = 96.95 %

### Example-12

### Preparation of 2-chloro 1,3-(bis piperidinyl) trimethinium hexafluoro phosphate

N-formyl piperidine (30g) was cooled to 0 to 10 °C and Chloroacetyl chloride (30g) was added drop wise at 0 to 10 °C. Subsequently, phosphorous oxychloride (6.5g) was added drop wise at 0 to 10 °C. Further, N-formyl piperidine (30g) was added drop wise at 0-10 °C and reaction mixture was maintained at 80-90 °C for 16 hrs. Then the reaction mixture was cooled to 25-30 °C and diluted with DMF. Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
Yield = 55.3 g, (54 %), HPLC purity = 72.74 %.

### Example-13

### Preparation of 2-chloro 1,3-(bis piperidinyl) trimethinium hexafluoro phosphate

Chloroacetyl chloride (10g) was cooled to 0 to 5 °C and N-formyl piperidine (10g) was added drop wise at 5 to 10 °C and reaction mixture was stirred for 1 hr. Subsequently, phosphorous oxychloride (13.6g) was added drop wise at 0 to 10 °C and further N-formyl piperidine (10g) was added at 0-10 °C, stirred for 1 hrs at 0-5 °C. The reaction mixture was maintained at 75-80 °C for 19 hrs. Then the reaction mixture was cooled to 25-30 °C and diluted with DMF. Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
Yield = 7.6 g, (22.22 %), HPLC purity = 80.33 %

### Example-14

### Preparation of 2-chloro 1,3-(bis piperidinyl) trimethinium hexafluoro phosphate

Chloroacetyl chloride (4.9g) was cooled to -5 to 0 °C and phosphorous oxychloride (6.7g) was added in one lot at 0 to -5 °C and stirred for 5minutes. Subsequently, N-formyl piperidine (8g) was added drop wise at 0 to 5 °C and further stirred for 10 minutes at 25-30 °C. The reaction mixture was maintained at 90-100 °C for 6 hrs. Then after reaction mixture was cooled to 25-30 °C and diluted with DMF. Subsequently, the reaction mass was added into the solution of NaPF₆ within 60 minutes at 0-10 °C and stirred for 60 minutes at 0-10 °C. The solid was precipitated out, which was filtered and washed with cold methanol: water to obtain 2-chloro 1,3-(bis piperidinyl) trimethinium hexafluoro phosphate salt.
Yield = 10.5 g, (51.19 %), HPLC purity = 83.87 %.

### Example-15

### Preparation of 2-chloro 1,3-(bis piperidinyl) trimethinium hexafluoro phosphate

N-formyl piperidine (20g) was cooled to 0 to -5 °C and chloroacetyl chloride (10g) was added simultaneously, phosphorous oxychloride (13.4g) was added drop wise at -5 to 5 °C and the reaction mixture was stirred at 25-30 °C for 10 minutes. The reaction mixture was maintained at 90-100 °C for 6 hrs. Then the reaction mixture was cooled to 25-30 °C and diluted with DMF. Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
Yield = 20 g, (47.39 %), HPLC purity = 81.05 %.

### Example-16 (Reference Example)

### Preparation of 2-chloro 1,3-(bis piperidinyl) trimethinium hexafluoro phosphate

Chloroacetic acid (4.75 g, 0.05 mole) was added into N-formyl piperidine (13.5 g, 0.119 mole) at 25-30 °C and heat the reaction mixture at 70-75 °C. Subsequently phosphorous oxychloride (9.5 ml, 0.1 mole) was added over a period of 2 hrs at 70-75 °C. The reaction mixture was maintained at 70-80 °C for 3 hrs. Then after reaction mixture was cooled to 25-30 °C and diluted with absolute alcohol (10 ml). Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
Yield = 2 g, (10.42 %), HPLC purity = 83.63 %.

### Example-17

### Preparation of 2-chloro 1,3-(bis piperidinyl) trimethinium hexafluoro phosphate

Chloroacetyl chloride (20g) was cooled at -10 to 0 °C and phosphorous oxychloride (27.2g)was added in one lot at -10 to -15 °C and simultaneously N-formyl piperidine (40g) was added drop wise at -8 to -10 °Cover a period of 1 hr. The reaction mixture was maintained at 90-100 °C for 6 hrs. Then after reaction mixture was cooled to 25-30 °C and diluted with absolute alcohol (10 ml). Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
Yield = (45.27 %), 37.2 g, HPLC purity = 83.24 %.

### Example-18

### 1^{st} Purification of 2-chloro 1,3-(bis piperidinyl) trimethinium hexafluoro phosphate

2-chloro 1,3-(bis piperidyl) trimethinium hexafluoro phosphate (37g) was dissolved in (185ml) methanol and (18.5ml) water (5:0.5) at 25-30 °C. The reaction mixture was stirred at 70-75 °C for 1 hrs. The reaction mixture was cooled to 0-10 °C and further stirred for 1 hrs, reaction mass was filtered and washed with methanol: water (1:1).
Yield = 23 g, (74.67 %), HPLC purity = 97.25 %.

### Example-19

### 2^{nd} Purification of 2-chloro 1,3-(bis piperidinyl) trimethinium hexafluoro phosphate

2-chloro 1,3-(bis piperidyl) trimethinium hexafluoro phosphate (11.2g) was dissolved in (55ml) methanol and aq. solution of bisulphate at 25-30 °C. The reaction mixture was stirred at 25-30 °C for 1 hrs, reaction mass was filtered and washed with water to obtain highly pure 2-chloro 1,3-(bis piperidinyl) trimethinium hexafluoro phosphate. Yield = 9.4 g, (83.92 %), HPLC purity = 99.19 %.

### Example-20

### Preparation of Etoricoxib

Potassium tert. butoxide (2.1g) was dissolved in THF (20ml) and the solution was maintained at 25-30 °C for 10 minutes and then cooled to 10-15 °C. Subsequently, solution of 1-(6-methylpyridin-3-yl)-2[4-(methylsulfonyl)phenyl] ethanone (5g) in THF was added and kept the mixture for 1hr at 25-30 °C. Subsequently, 2-chloro 1,3-(bis piperidinyl) trimethinium hexafluoro phosphate salt (8g), in THF (40ml) was added and maintained the reaction mixture for 2 hrs at 25-30 °C. The reaction mixture was dumped in to the mixture of trifloroacetic acid (1g), acetic acid (7.5g), and THF (100ml) and maintained for 1 hour at 25-30 °C. Further the reaction mixture was heated at 55-65 °C for 17 hrs after aq. ammonia solution (125 ml) was added and the reaction mixture was cooled to 25-30 °C. After that ethyl acetate and water was added, organic layer was separated and washed with water and 10 % sodium bicarbonate solution. Organic layer was separated, dried and concentrated to dryness at less than 50 °C to give crude Etoricoxib.
Yield = 8.6 g, HPLC purity = 91.28 % (Etoricoxib) (unreacted sulfone compound < 5 %).

### Example-21

### Preparation of Etoricoxib

Potassium tert. butoxide (11.6g) was dissolved in THF (90ml) and the solution was maintained at 25-30 °C for 10 minutes and then cooled to 10-15 °C. Subsequently, solution of 1-(6-methylpyridin-3-yl)-2[4-(methylsulfonyl)phenyl] ethanone (27.5g) in THF was added and kept the mixture for 1hr at 15-25 °C. Subsequently, 2-chloro 1,3-(bis piperidinyl) trimethinium hexafluoro phosphate salt (44g), in THF (220ml) was added and maintained the reaction mixture for 3.5 hrs at 25-30 °C. The reaction mixture was dumped in to the mixture of trifloroaceticacid (5.5g), acetic acid (41.2g), and THF (65ml) and maintained for 1 hour at 25-30 °C. Further the reaction mixture was heated at 55-65 °C for 17 hrs after aq. ammonia solution (190ml) was added and the reaction mixture was cooled to 25-30 °C. Then ethyl acetate and water was added, organic layer was separated and washed with water and 10 % sodium bicarbonate solution. Organic layer was separated, dried and concentrated to dryness < 50 °C to give crude Etoricoxib.
Yield = 48.7 g, HPLC purity = 94.45 % (Etoricoxib), (unreacted sulfone compound < 5 %).

### Example-22

### 1^{st} Purification of Etoricoxib

(48.5 g) crude Etoricoxib (purity = 94.45 %, sulfone compound < 5%) obtained above was dissolved in 200 ml methanol and subsequently, (27.5g) formalin solution (37 %) and (27.5ml) aq. ammonia solution were added at 25-30 °C. The reaction mixture was stirred at 45-55 °C for 2 hrs and then remove the solvent under reduced pressure at < 55 °C. 250 ml ethyl acetate was added and reaction mixture was stirred. Organic layer was separated and washed with water, charcolized with (2.7g) charcoal, filtered and concentrated at < 55 °C, azeotrope with 50 ml isopropyl alcohol to dryness.
Yield = 34.5 g (97.9 %), HPLC purity = 96.49 % (Etoricoxib), (unreacted sulfone compound < 1 %).

### Example-23

### 2^{nd} Purification of Etoricoxib

(34.4 g) crude Etoricoxib (purity = 96.49 %) was stirred in isopropyl alcohol at 60-65 °C for 1 hr. The reaction mixture was cooled to 25-30 °C and further stirred for 17 hrs. Further (27.5 ml) isopropyl alcohol was added and stirred for 10 minutes at 25-30 °C, filter and washed with isopropyl alcohol, material was dried at 50-55 °C.
Yield = 23.3 g, (69.28 %), HPLC purity > 98 % Etoricoxib.

### Example-24

### Preparation of Etoricoxib

Potassium tert. butoxide (0.4g) was dissolved in DMF (4ml) and the solution was maintained at 25-30 °C for 10 minutes and then cooled to 10-15 °C. Subsequently, solution of 1-(6-methylpyridin-3-yl)-2[4-(methylsulfonyl)phenyl] ethanone (1g) in DMF was added and kept the mixture for 1hr at 15-25 °C. Subsequently, 2-chloro 1,3-(bis piperidinyl) trimethinium hexafluoro phosphate salt (1.6g), in DMF (16ml) was added and maintained the reaction mixture for 2 hr at 25-30 °C. The reaction mixture was dumped in to the mixture of trifloroacetic acid (0.2g), acetic acid (1.5g), and DMF (12ml) and maintained for 1 hour at 25-30 °C. Further the reaction mixture was heated at 55-65 °C for 18 hrs after ammonia solution (25ml) was added and the reaction mixture was cooled to 25-30 °C. After then ethyl acetate and water was added, organic layer was separated and washed with water and 10 % sodium bicarbonate solution. Organic layer was separated, dried and concentrated to dryness < 50 °C to give crude Etoricoxib.
Yield = (55.12 %) 1 g, HPLC purity = 67.8 % (Etoricoxib), (unreacted sulfone compound < 5 %).

### Example-25

### Preparation of Etoricoxib

1-(6-methylpyridin-3-yl)-2[4-(methylsulfonyl)phenyl] ethanone (9g) was dissolved in THF and the solution was maintained at 25-30 °C for 10 minutes and then cooled to 10-15 °C. Subsequently, potassium tert. butoxide (4.1 g) was added and reaction mixture was maintained at 10-15 °C for 60 minutes. After then was added 2-chloro 1,3-(bis piperidinyl) trimethinium hexafluoro phosphate (14.8 g), followed by THF. The reaction mixture was maintained for 2 hrs at 10-15 °C and the reaction mixture was dumped into acetic acid (13 g) at 10-15 °C, washed with THF. Subsequently, the reaction mixture was maintained for 2 hour at 10-15 °C, further the reaction mixture was maintained at 60-65 °c for 16 hrs after adding aq. ammonia solution 25 ml and ammonium acetate (2.5 g). The reaction mixture was cooled to 25-30 °C, organic layer was separated and 25g aq. ammonia solution and 2 g formaldehyde solution was added at 25-30 °C. The reaction mixture was maintained at 60-65 °C for 1 hrs, cooled to 40 °C and 2.7 g sodium chloride was added in it & stirred for 30 minutes. Organic layer was separated and distilled till complete solvent removal under reduced pressure. The residue was azeotroped with toluene, and washed with water at 70-75 °C, aqueous layer was separated and extracted with toluene followed by toluene at 70-75 °C. Combined all the organic layers and washed with 7.5 % sodium bicarbonate solution followed by water. Further organic layer was separated and charcolized with 0.3 g charcoal at 60-65 °C, and separated organic layer was distilled under reduced pressure, residue was azeotroped with IPA at 25-30 °C. The reaction mixture was stirred at 75-80 °C for 30 minutes, cooled to 25-30 °C, further IPA was added at 25-30 °C, cooled to 10-15 °C, stirred for 2 hrs at 10-15 °C, filtered and washed with cold IPA.
Yield = 6.8 g (61 %), HPLC purity = 99.34 % (Etoricoxib), ( unreacted sulfone compound < 5 %).

### Example-26

### Purification of Etoricoxib

Etoricoxib (5 g) (as obtained in example 25) was dissolved in isopropyl alcohol and stirred at 60-65 °C for 1 hrs after adding 0.17 g charcoal, filtered through hyflow & washed with 2 ml hot IPA. The reaction mixture was cooled to 25-30 °C and stirred for 2 hrs, filtered and washed with isopropyl alcohol, dried the material at 60-65 °C to obtain pure Etoricoxib.
Yield = 3.6 g (72.87 %), HPLC purity 99.83 % Etoricoxib.
Etoricoxib obtained is also characterized by an XPRD peaks at 7.00, 8.50, 9.67, 11.72, 12.35, 12.96, 13.24, 15.04, 15.44, 16.51, 17.06, 17.70, 18.07, 18.76, 19.34, 20.00, 20.24, 21.14, 21.50, 22.20, 22.70, 23.28, 24.02, 24.76, 25.73, 26.20, 26.90, 27.37, 28.44, 29.24, 29.83, 30.41, 30.66, 31.22, 33.04, 34.63, 35.76, 37.62, 39.16, 39.65° ± 0.2° degrees (2θ)( Fig.1) and has melting point in the range of 135-137 °C.

### Example-27

### Preparation of 2-chloro 1,3-(bis piperidinyl) trimethinium hexafluoro phosphate

Chloroacetyl chloride (200g) was cooled at -10 to 0 °C and phosphorous oxychloride (271.5g) was added in one lot at 0 to -10 °C and simultaneously N-formyl piperidine (400g) was added drop wise at 0 to 10 °C over a period of 1 hr. The reaction mixture was maintained at 90-100 °C for 6 hrs. Then after reaction mixture was cooled to 25-30 °C and diluted with DMF. Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
Yield = 581.3 g (78.7 %), HPLC purity = 92.81 %.

### Example-28

### 1^{st} Purification of 2-chloro 1,3-(bis piperidinyl) trimethinium hexafluoro phosphate

2-chloro 1,3-(bis piperidinyl) trimethinium hexafluoro phosphate (580 g) (as obtained in example 27) was dissolved in a mixture of methanol and water (5:0.5) at 25-30 °C. The reaction mixture was stirred at 60-70 °C for 1.5 hrs, cooled to 0-10 °C. Further, the reaction mixture was stirred for 1 hrs, filtered and washed with methanol: water (1:1) to obtain pure 2-chloro 1,3-(bis piperidinyl) trimethinium hexafluoro phosphate.
Yield = 340.5 g (63.28 %), HPLC purity = 99.57 %.

### Example-29

### 2^{nd} Purification of 2-chloro 1,3-(bis piperidinyl) trimethinium hexafluoro phosphate

2-chloro 1,3-(bis piperidinyl) trimethinium hexafluoro phosphate (340.5g) was dissolved in methanol and aq. solution of bisulphate at 25-30 °C. The reaction mixture was stirred at 25-30 °C for 1 hr, reaction mass was filtered and washed with water to obtain highly pure 2-chloro 1,3-(bis piperidinyl) trimethinium hexafluoro phosphate.
Yield = 305 g, (90.15 %), HPLC purity = 99.65 %.
2-chloro 1,3-(bis piperidinyl) trimethinium hexafluoro phosphate obtained is also characterized by an XPRD peaks at 9.061, 11.60, 12.06, 12.89, 13.64, 14.38, 16.022, 16.98, 17.56, 18.16, 19.43, 20.16, 20.46, 21.04, 21.48, 21.98, 22.25, 22.68, 23.35, 23.76, 24.24, 24.64, 25.92, 26.75, 27.42, 28.03, 28.55, 29.04, 29.68, 30.06, 30.52, 31.25, 31.96, 32.36, 33.31, 33.94, 34.30, 35.16, 35.80, 38.14, 39.47 ° ± 0.2 ° degrees (2θ)( Fig. 2, Form I) and has melting point in the range of 185-200 °C. (Decomposed).

### Example-30

### Preparation of 2-chloro 1,3-(bis piperidinyl) trimethinium hexafluoro phosphate

Chloroacetyl chloride (100g) was cooled to 0 °C and phosphorous oxychloride (135.7g) was added in one lot at 0 to -10 °C and simultaneously N-formyl piperidine (200g) was added drop wise at 0 to 10 °C over a period of 1 hr. The reaction mixture was maintained at 90-100 °C for 6 hrs. Then the reaction mixture was cooled to 25-30 °C and diluted with DMF. Subsequently, the reaction mass was added into the solution of NaPF₆ (prepared by adding 5N aq. NaOH solution in aq. HPF₆ solution) within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
Yield = 189.3 g (45.9 5), HPLC purity: 82.41 %.

### Example-31

### 1^{st} Purification of 2-chloro 1,3-(bis piperidinyl) trimethinium hexafluoro phosphate

2-chloro 1,3-(bis piperidyl) trimethinium hexafluoro phosphate (180 g) (as obtained in example 30) was dissolved in a mixture of methanol and water (5:0.5) at 25-30 °C. The reaction mixture was stirred at 60-70 °C for 1.5 hrs, cooled to 0-10 °C. Further, the reaction mixture was stirred for 1 hr, filtered and washed with methanol: water (1:1) to obtain pure 2-chloro 1,3-(bis piperidinyl) trimethinium hexafluoro phosphate.
Yield = 110 g (73.36 %), HPLC purity: 99.06 %

### Example-32

### 2^{nd} Purification of 2-chloro 1, 3-(bis piperidinyl) trimethinium hexafluoro phosphate

2-chloro 1,3-(bis piperidyl) trimethinium hexafluoro phosphate (109.6g; obtained in Example 31) was dissolved in methanol and aq. solution of bisulphate (29.5 g sodium bisulphite dissolved in 109.6 ml water) at 25-30 °C. The reaction mixture was stirred at 25-30 °C for 1 hr, reaction mass was filtered and washed with water to obtain highly pure 2-chloro 1,3-(bis piperidinyl) trimethinium hexafluoro phosphate.
Yield = 91.9 g (84.65 %), HPLC purity: 99.51 %.
2-chloro 1,3-(bis piperidinyl) trimethinium hexafluoro phosphate obtained is also characterized by an XPRD peaks at 9.14, 10.142, 12.12, 12.96, 13.71, 17.06, 17.64, 18.24, 19.49, 20.520, 21.10, 21.56, 22.32, 22.74, 23.42, 24.32, 24.72, 26.05, 27.48, 28.10, 29.14, 29.79, 30.12, 30.61, 31.30, 32.06, 32.43, 33.36, 34.02, 34.38 ° ± 0.2 ° degrees (2θ)( Fig. 3, Form II) and has melting point in the range of 190-197 °C.

## Claims

1. A process for the preparation of Etoricoxib of formula-(I) comprising, 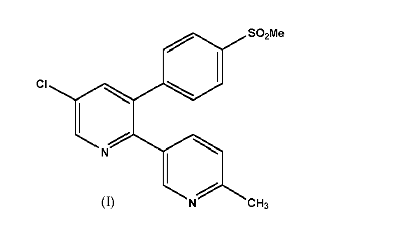
(a) reacting a compound of formula-(IV) with suitable chloroacetyl halide or chloro acetic acid and phosphorous oxychloride to obtain the compound of formula (IIId) & further converting the compound of formula (IIId) into its suitable salt to obtain the compound of formula-(IIIb), 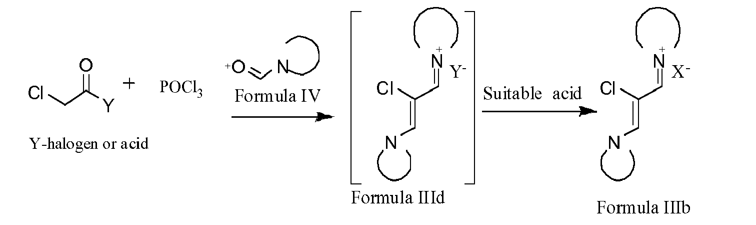 wherein 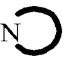 represents a cyclic group, optionally containing one or more heteroatoms selected from N, O or S & 'X⁻' represents a suitable counter ion.
(b) reacting the compound of formula (IIIb) with compound of formula (II) in presence of suitable base and suitable solvent to obtain Etoricoxib of formula (I)

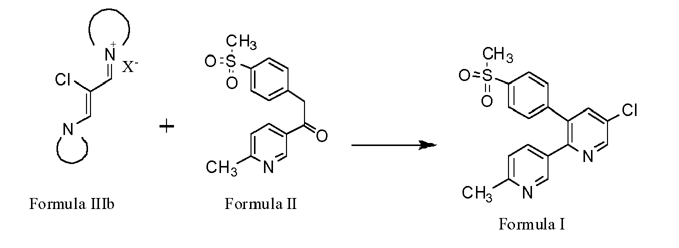

2. The process as claimed in claim 1, wherein 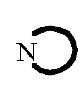 is selected from morpholidinyl, piperidinyl and pyrolidinyl group.

3. The process as claimed in 1, wherein suitable salt of compound of formula (IIIb) is prepared by reaction of the compound of formula (IIId) with a suitable salt of an acid to obtain corresponding salt of compound formula (IIIb).

4. The process as claimed in claim 3, wherein the suitable salt of an acid is selected from the group consisting of phosphate, sulphate, acetates, perchlorate, borates, antimonite, halides, benzoate, napsylate, hexafluorophosphate, tetrafluoro borate, chloride, bromide, fluoride, iodide, benzoate and carbonate.

5. The process as claimed in claim 4, wherein the suitable salt of acid is hexafluorophosphate.

6. The process as claimed in claim 1 in step (b), wherein the suitable solvent is selected from C₁-C₆ alcohols, hydrocarbons selected from benzene, toluene and xylene; halocarbon solvents selected from mono or di halo C₁-C₄ alkyls, nitrogen containing solvents selected from DMF, dimethyl acetamide, N-ethylpyrrolidinone, N-methyl pyrrolidinone and acetonitrile and dioxane and dimethyl sulfoxide.

7. The process as claimed in claim 6 wherein the solvents are selected from C₁-C₆ alcohol, THF, DMF, dioxane and dimethyl sulfoxide.

8. The process as claimed in claim 1 step (b), wherein the suitable base used is selected from suitable organic or inorganic bases, wherein the inorganic bases are selected from alkali metal or alkaline earth metal hydroxides, alkali metal or alkaline earth metal carbonates such as NaHCO₃, Na₂CO₃, K₂CO₃, alkali metal hydrides, alkali metal alkoxides.

9. A process for preparation of the compound of formula-(IIIb), wherein, 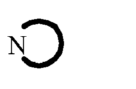 represents a cycloalkyl group, optionally containing one more heteroatom selected from N, O or S & 'X⁻' represents the suitable counterion, as claimed in claim 1, comprising 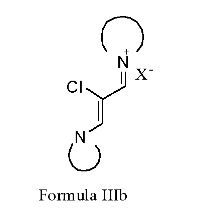 reacting a compound of formula-(IV) with suitable chloroacetyl halide or chloro acetic acid and phosphorous oxychloride by combining the chloroacetyl halide or chloro acetic acid and phosphorous oxychloride at room temperature or below, and adding the compound of formula (IV) to the combination to obtain the compound of formula (IIId) & further converting the compound of formula (IIId) into its suitable salt of formula-(IIIb),

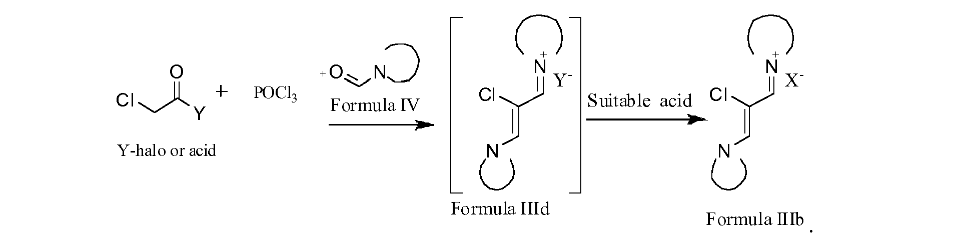

10. The process as claimed in claim 9, wherein the temperature is less than 30 °C.

11. The process as claimed in claim 9, wherein the reaction mixture, after addition of the reactants, is heated to a temperature of 50° to 150 °C, more preferably at 75 °C to 120 °C and most preferably, at 90 °C to 100 °C, to obtain the compound of formula (IIIb).

12. The process as claimed in claim 9, wherein the reaction is carried out for 3 hours to 25 hours, preferably for 6 to 8 hrs.

13. The process of claim 9, wherein the compound of formula (IIId) is converted to its suitable salt of formula (IIIb) by reacting it with the salt of corresponding acids.

14. The process as claimed in claim 13 wherein, the suitable salt of acid is selected from the group comprising of phosphate, sulphate, acetates, perchlorate, borates, antimonite, halides, benzoate, napsylate such as hexafluorophosphate, tetrafluoro borate, chloride, bromide, fluoride, iodide, benzoate, carbonate, hexafluoroantimonate.

15. The process as claimed in claim 14, wherein the suitable salt of acid is hexafluorophosphate.

16. The process as claimed in claim 9, wherein the compound of formula (IIIb) is further purified by
i) dissolving the compound of formula (IIIb) in C₁-C₅ alcohols or their esters, optionally with water & subsequently isolating the compound of formula (IIIb);
ii) treating the compound of formula (IIIb) with suitable alkali bisulphite or metabisulphite, in C₁-C₅ alcohols or their esters and subsequently isolating the pure compound of formula (IIIb).

17. The process as claimed in claim 16, wherein the alkali metal bisulphite or metabisulphite is selected from sodium or potassium bisulphite or metabisulphite.

18. The process as claimed in claim 16, wherein the salt of formula (IIIb) is obtained with at least 99 % purity by HPLC.

19. A compound of structural formula-(IIIc), wherein 'X⁻' represents a suitable counter ion.

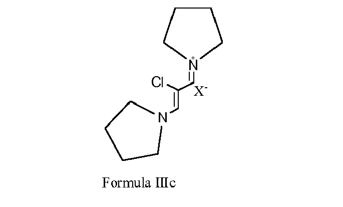

20. Polymorphic form I of 2-chloro 1,3-(bis piperidinyl) trimethinium hexafluoro phosphate, **characterized by** a PXRD pattern with peaks at 9.06, 11.60, 12.06, 12.89, 13.64, 14.38, 16.02, 16.98, 17.56, 18.16, 19.43, 20.16, 20.46, 21.04, 21.48, 21.98, 22.25, 22.68, 23.35, 23.76, 24.24, 24.64, 25.92, 26.75, 27.42, 28.03, 28.55, 29.04, 29.68, 30.06, 30.52, 31.25, 31.96, 32.36, 33.31, 33.94, 34.30, 35.16, 35.80, 38.14, 39.47 ° ± 0.2 ° degrees (2θ).

21. Polymorphic form II of 2-chloro 1,3-(bis piperidinyl) trimethinium hexafluoro phosphate, **characterized by** a PXRD pattern with peaks at 9.14, 10.142, 12.12, 12.96, 13.71, 17.06, 17.64, 18.24, 19.49, 20.520, 21.10, 21.56, 22.32, 22.74, 23.42, 24.32, 24.72, 26.05, 27.48, 28.10, 29.14, 29.79, 30.12, 30.61, 31.30, 32.06, 32.43, 33.36, 34.02, 34.38 ° ± 0.2 ° degrees (2θ).

## Patentansprüche

1. Verfahren zur Herstellung von Etoricoxib der Formel (I), umfassend 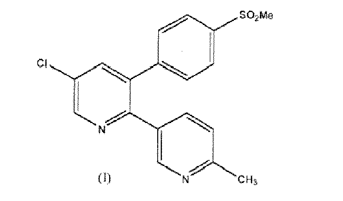
(a) Umsetzung einer Verbindung der Formel (IV) mit geeignetem Chloracetylhalogenid oder Chloressigsäure und Phosphoroxychlorid, um die Verbindung der Formel (IIId) zu erhalten und weitere Überführung der Verbindung der Formel (IIId) in deren geeignetes Salz, um die Verbindung der Formel (IIIb) zu erhalten, 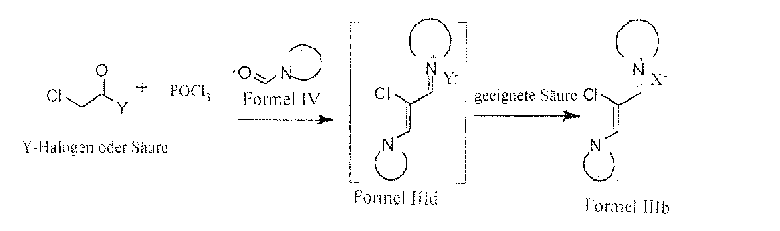 worin 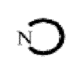 eine cyclische Gruppe darstellt, gegebenenfalls enthaltend ein oder mehrere Heteroatom(e), ausgewählt aus N, O oder S und ,X⁻'ein geeignetes Gegenion darstellt,
(b) Umsetzung der Verbindung der Formel (IIIb) mit einer Verbindung der Formel (II) in Gegenwart einer geeigneten Base und von geeignetem Lösungsmittel, um Etoricoxib der Formel (I) zu erhalten

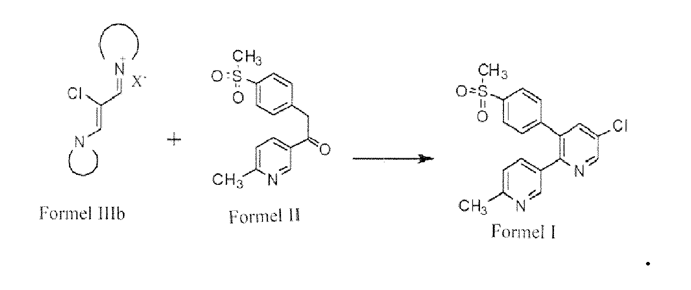

2. Verfahren wie in Anspruch 1 beansprucht, worin 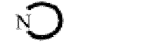 ausgewählt ist aus einer Morpholidinyl-, Piperidinyl- und Pyrrolidinylgruppe.

3. Verfahren wie in Anspruch 1 beansprucht, wobei das geeignete Salz der Verbindung der Formel (IIIb) hergestellt wird durch Umsetzung der Verbindung der Formel (IIId) mit einem geeigneten Salz, einer Säure, um das entsprechende Salz der Verbindung der Formel (IIIb) zu erhalten.

4. Verfahren wie in Anspruch 3 beansprucht, wobei das geeignete Salz einer Säure ausgewählt ist aus der Gruppe, bestehend aus Phosphat, Sulfat, Acetaten, Perchlorat, Boraten, Antimonit, Halogeniden, Benzoat, Napsylat, Hexafluorphosphat, Tetrafluorborat, Chlorid, Bromid, Fluorid, Iodid, Benzoat und Carbonat.

5. Verfahren wie in Anspruch 4 beansprucht, wobei das geeignete Salz der Säure Hexafluorphosphat ist.

6. Verfahren wie in Anspruch 1 in Schritt (b) beansprucht, wobei das geeignete Lösungsmittel ausgewählt ist aus C₁-C₆-Alkoholen, Kohlenwasserstoffen, ausgewählt aus Benzol, Toluol und Xylol, Halogenkohlenstofflösungsmitteln, ausgewählt aus Mono- oder Di-Halogen-C₁-C₄-Alkylen, Stickstoff-enthaltenden Lösungsmitteln, ausgewählt aus DMF, Dimethylacetamid, N-Ethylpyrrolidinon, N-Methylpyrrolidinon und Acetonitril, und Dioxan und Dimethylsulfoxid.

7. Verfahren wie in Anspruch 6 beansprucht, wobei die Lösungsmittel ausgewählt sind aus C₁-C₆-Alkohol, THF, DMF, Dioxan und Dimethylsulfoxid.

8. Verfahren wie in Anspruch 1 (Schritt (b)) beansprucht, wobei die verwendete geeignete Base ausgewählt ist aus geeigneten organischen oder anorganischen Basen, wobei die anorganischen Basen ausgewählt sind aus Alkalimetall- oder Erdalkalimetallhydroxiden, Alkalimetall- oder Erdalkalimetallcarbonaten, wie NaHCO₃, Na₂CO₃, K₂CO₃, Alkalimetallhydriden, Alkalimetalloxiden.

9. Verfahren zur Herstellung der Verbindung der Formel (IIIb), wobei 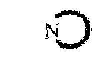 eine Cycloalkylgruppe darstellt, gegebenenfalls enthaltend eines oder mehrere Heteroatom(e), ausgewählt aus N, O oder S und ,X^{-'} ein geeignetes Gegenion darstellt, wie in Anspruch 1 beansprucht, umfassend 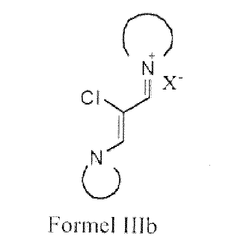 die Umsetzung einer Verbindung der Formel (IV) mit geeignetem Chloracetylhalogenid oder Chloressigsäure und Phosphoroxychlorid durch Kombination des Chloracetylhalogenids oder der Chloressigsäure und Phosphoroxychlorid bei Raumtemperatur oder darunter, und Zugabe der Verbindung der Formel (IV) zu der Kombination, um die Verbindung der Formel (IIId) zu erhalten, und weitere Überführung der Verbindung der Formel (IIId) in deren geeignetes Salz der Formel (IIIb),

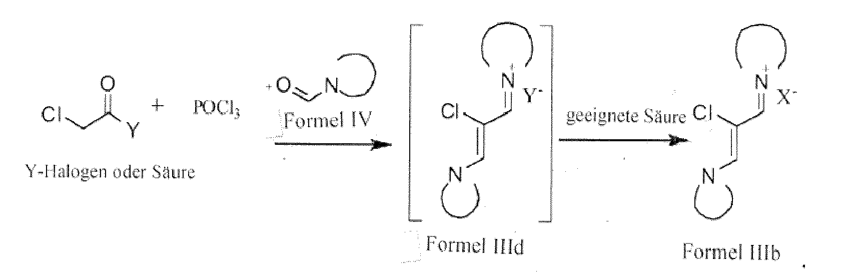

10. Verfahren wie in Anspruch 9 beansprucht, wobei die Temperatur weniger als 30°C beträgt.

11. Verfahren wie in Anspruch 9 beansprucht, wobei das Reaktionsgemisch, nach der Zugabe der Reaktanten, auf eine Temperatur von 50°C bis 150°C, bevorzugt auf 75°C bis 120°C und insbesondere auf 90°C bis 100°C erhitzt wird, um die Verbindung der Formel (IIIb) zu erhalten.

12. Verfahren wie in Anspruch 9 beansprucht, wobei die Umsetzung ausgeführt wird für 3 bis 25 Stunden, vorzugsweise 6 bis 8 Stunden.

13. Verfahren nach Anspruch 9, wobei die Verbindung der Formel (IIId) überführt wird in deren geeignetes Salz der Formel (IIIb) durch deren Umsetzung mit dem Salz der korrespondierenden Säuren.

14. Verfahren wie in Anspruch 13 beansprucht, wobei das geeignete Salz der Säure ausgewählt ist aus der Gruppe, umfassend Phosphat, Sulfat, Acetate, Perchlorat, Borate, Antimonit, Halogenide, Benzoat, Napsylat, wie Hexafluorphosphat, Tetrafluorborat, Chlorid, Bromid, Fluorid, Iodid, Benzoat, Carbonat, Hexafluorantimonat.

15. Verfahren wie in Anspruch 14 beansprucht, wobei das geeignete Salz der Säure Hexafluorphosphat ist.

16. Verfahren wie in Anspruch 9 beansprucht, wobei die Verbindung der Formel (IIIb) weiter gereinigt wird durch
i) Auflösen der Verbindung der Formel (IIIb) in C₁-C₅-Alkoholen oder deren Estern, gegebenenfalls mit Wasser und nachfolgende Isolierung der Verbindung der Formel (IIIb);
ii) Behandeln der Verbindung der Formel (IIIb) mit geeignetem Alkalibisulfit oder -metabisulfit in C₁-C₅-Alkoholen oder deren Estern und nachfolgende Isolierung der gereinigten Verbindung der Formel (IIIb).

17. Verfahren wie in Anspruch 16 beansprucht, wobei das Alkalimetallbisulfit oder -metabisulfit ausgewählt ist aus Natrium- oder Kaliumbisulfit oder -metabisulfit.

18. Verfahren wie in Anspruch 16 beansprucht, wobei das Salz der Formel (IIIb) in einer Reinheit von mindestens 99% nach HPLC erhalten wird.

19. Verbindung der Strukturformel (IIIc), worin ,X⁻' ein geeignetes Gegenion darstellt.

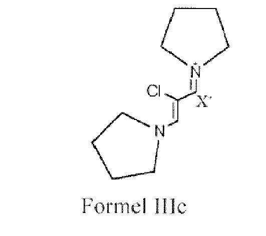

20. Polymorphe Form I von 2-Chlor-1,3-(bis-piperidinyl)trimethiniumhexafluorphosphat, **gekennzeichnet durch** ein PXRD-Muster mit Peaks bei 9,06, 11,60, 12,06, 12,89, 13,64, 14,38, 16,02, 16,98, 17,56, 18, 16, 19,43, 20,16, 20,46, 21,04, 21,48, 21,98, 22,25, 22,68, 23,35, 23,76, 24,24, 24,64, 25,92, 26,75, 27,42, 28,03, 28,55, 29, 04, 29, 68, 30, 06, 30,52, 31,25, 31, 96, 32,36, 33,31, 33,94, 34,30, 35,16, 35,80, 38,14, 39,47° ± 0,2° Grad (2θ).

21. Polymorphe Form II von 2-Chlor-1,3-(bis-piperidinyl)trimethiniumhexafluorphosphat, **gekennzeichnet durch** ein PXRD-Muster mit Peaks bei 9,14, 10,142, 12,12, 12,96, 13,71, 17,06, 17,64, 18,24, 19,49, 20,520, 21,10, 21,56, 22,32, 22,74, 23,42, 24,32, 24,72, 26,05, 27,48, 28, 10, 29,14, 29,79, 30, 12, 30,61, 31,30, 32, 06, 32,43, 33,36, 34,02, 34,38° ± 0,2° Grad (2θ).

## Revendications

1. Procédé de préparation de l'étoricoxib, de formule (I) 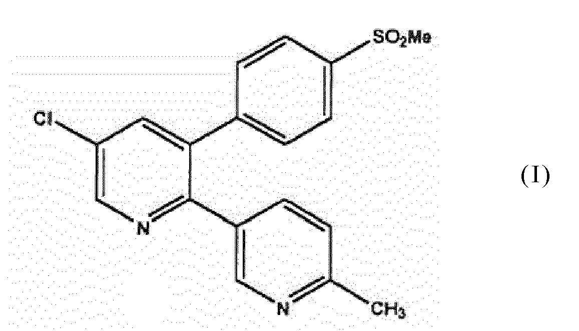 lequel procédé comporte les étapes suivantes :
a) faire réagir un composé de formule (IV) avec un halogénure de chloro-acétyle approprié ou de l'acide chloro-acétique et de l'oxychlorure de phosphore, de manière à obtenir un composé de formule (IIId), et convertir ensuite ce composé de formule (IIId) en un sel approprié, pour obtenir un composé de formule (IIIb) : 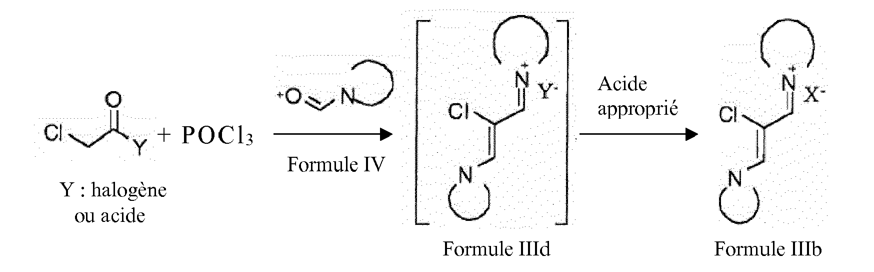 où 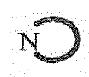 représente un groupe cyclique qui, en option, comporte un ou plusieurs hétéroatome(s) choisi(s) parmi les atomes d'azote, d'oxygène ou de soufre, et X⁻ représente un contre-ion approprié ;
b) et faire réagir le composé de formule (IIIb) avec le composé de formule (II), en présence d'une base appropriée et dans un solvant approprié, pour obtenir l'étoricoxib de formule (I) :

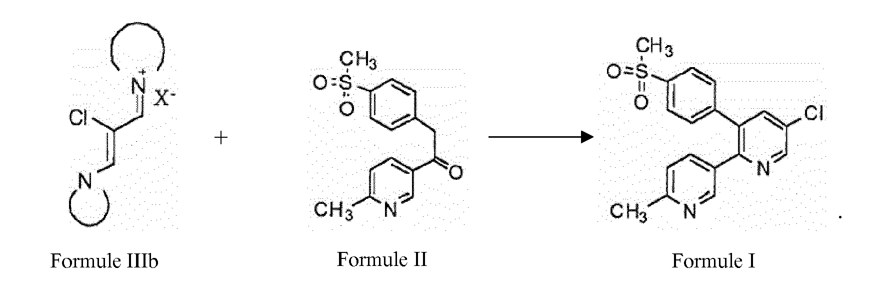

2. Procédé conforme à la revendication 1, dans lequel 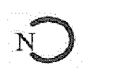 représente un groupe choisi parmi les groupes morpholinyle, pipéridinyle et pyrrolidinyle.

3. Procédé conforme à la revendication 1, dans lequel on prépare le sel approprié du composé de formule (IIIb) en faisant réagir le composé de formule (IIId) avec un sel d'acide approprié, pour obtenir le sel correspondant du composé de formule (IIIb).

4. Procédé conforme à la revendication 3, dans lequel le sel d'acide approprié est choisi dans l'ensemble constitué par les phosphate, sulfate, acétates, perchlorate, borates, antimonite, halogénures, benzoate, napsylate, hexafluorophosphate, tétrafluoroborate, chlorure, bromure, fluorure, iodure, benzoate et carbonate.

5. Procédé conforme à la revendication 4, dans lequel le sel d'acide approprié est un hexafluorophosphate.

6. Procédé conforme à la revendication 1, dans lequel, dans l'étape (b), le solvant approprié est choisi parmi des alcools en C₁-C₆, des hydrocarbures choisis parmi les benzène, toluène et xylène, des solvants halogéno-carbonés choisis parmi les alcanes en C₁-C₄ mono-halogénés ou dihalogénés, des solvants azotés choisis parmi les DMF, diméthyl-acétamide, N-éthyl-pyrrolidone, N-méthyl-pyrrolidone et acétonitrile, et les dioxane et diméthyl-sulfoxyde.

7. Procédé conforme à la revendication 6, dans lequel le solvant est choisi parmi les suivants : alcools en C₁-C₆, THF, DMF, dioxane et diméthyl-sulfoxyde.

8. Procédé conforme à la revendication 1, dans lequel, dans l'étape (b), la base appropriée utilisée est choisie parmi des bases appropriées organiques ou inorganiques, lesquelles bases inorganiques sont choisies parmi les hydroxydes de métal alcalin ou de métal alcalino-terreux, les carbonates de métal alcalin ou de métal alcalino-terreux, tels NaHCO₃, Na₂CO₃ ou K₂CO₃, les hydrures de métal alcalin, et les alcoolates de métal alcalin.

9. Procédé de préparation d'un composé de formule (IIIb) 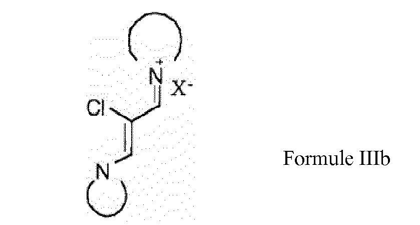 dans laquelle 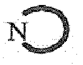 représente un groupe cycloalkyle qui, en option, comporte un et un seul hétéroatome supplémentaire choisi parmi les atomes d'azote, d'oxygène ou de soufre, et X⁻ représente un contre-ion approprié, tel que revendiqué dans la revendication 1,
lequel procédé comporte le fait de faire réagir un composé de formule (IV) avec un halogénure de chloro-acétyle approprié ou de l'acide chloro-acétique et de l'oxychlorure de phosphore, en combinant l'halogénure de chloro-acétyle ou l'acide chloro-acétique et l'oxychlorure de phosphore, à température ambiante ou inférieure, et en ajoutant le composé de formule (IV) à cette combinaison, de manière à obtenir un composé de formule (IIId), et le fait de convertir ensuite ce composé de formule (IIId) en le sel approprié de formule (IIIb) :

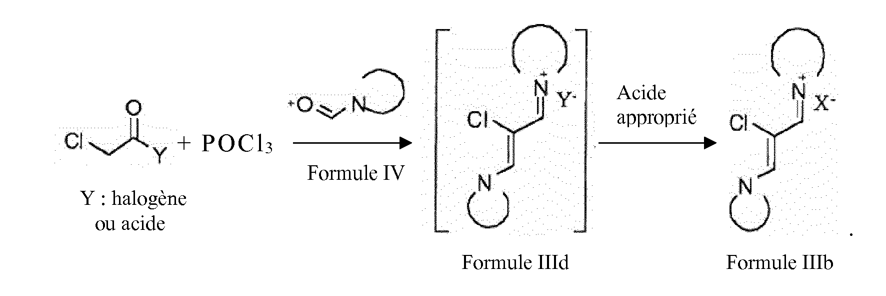

10. Procédé conforme à la revendication 9, dans lequel la température est inférieure à 30 °C.

11. Procédé conforme à la revendication 9, dans lequel, après addition des réactifs, on chauffe le mélange réactionnel à une température de 50 à 150 °C, de préférence de 75 à 120 °C et mieux encore de 90 à 100 °C, pour obtenir le composé de formule (IIIb).

12. Procédé conforme à la revendication 9, dans lequel la réaction est poursuivie durant 3 à 25 heures, et de préférence durant 6 à 8 heures.

13. Procédé conforme à la revendication 9, dans lequel on convertit le composé de formule (IIId) en le sel approprié de formule (IIIb) en le faisant réagir avec un sel de l'acide correspondant.

14. Procédé conforme à la revendication 13, dans lequel le sel d'acide approprié est choisi dans l'ensemble constitué par les phosphate, sulfate, acétates, perchlorate, borates, antimonite, halogénures, benzoate et napsylate, tels les hexafluorophosphate, tétrafluoroborate, chlorure, bromure, fluorure, iodure, benzoate, carbonate et hexafluoroantimonate.

15. Procédé conforme à la revendication 14, dans lequel le sel d'acide approprié est l'hexafluorophosphate.

16. Procédé conforme à la revendication 9, dans lequel on purifie en outre le composé de formule (IIIb) :
i) en dissolvant le composé de formule (IIIb) dans un alcool en C₁-C₅ ou un ester d'un tel alcool, en option avec de l'eau, et en isolant ensuite le composé de formule (IIIb) ;
ii) en traitant le composé de formule (IIIb) avec un bisulfite ou un métabisulfite de métal alcalin approprié, dans un alcool en C₁-C₅ ou un ester d'un tel alcool, et en isolant ensuite le composé de formule (IIIb), pur.

17. Procédé conforme à la revendication 16, dans lequel le bisulfite ou métabisulfite de métal alcalin est choisi parmi les bisulfites et métabisulfites de sodium ou de potassium.

18. Procédé conforme à la revendication 16, dans lequel on obtient le sel de formule (IIIb) avec une pureté d'au moins 99 % en CLHP.

19. Composé de formule structurale (IIIc), dans laquelle X⁻ représente un contre-ion approprié :

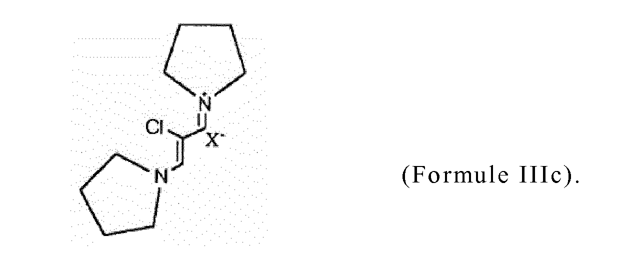

20. Forme polymorphique I de l'hexafluorophosphate de 2-chloro-1,3-bis(pipéridinyl)-triméthinium, **caractérisée par** un diagramme de diffraction des rayons X, à l'état de poudre, qui comporte des pics pour les valeurs suivantes de l'angle 2θ (à ± 0,2° près) : 9,06°, 11,60°, 12,06°, 12,89°, 13,64°, 14,38°, 16,02°, 16,98°, 17,56°, 18,16°, 19,43°, 20,16°, 20,46°, 21,04°, 21,48°, 21,98°, 22,25°, 22,68°, 23,35°, 23,76°, 24,24°, 24,64°, 25,92°, 26,75°, 27,42°, 28,03°, 28,55°, 29,04°, 29,68°, 30,06°, 30,52°, 31,25°, 31,96°, 32,36°, 33,31°, 33,94°, 34,30°, 35,16°, 35,80°, 38,14° et 39,47°.

21. Forme polymorphique II de l'hexafluorophosphate de 2-chloro-1,3-bis(pipéridinyl)-triméthinium, **caractérisée par** un diagramme de diffraction des rayons X, à l'état de poudre, qui comporte des pics pour les valeurs suivantes de l'angle 2θ (à ± 0,2° près) : 9,14°, 10,142°, 12,12°, 12,96°, 13,71°, 17,06°, 17,64°, 18,24°, 19,49°, 20,520°, 21,10°, 21,56°, 22,32°, 22,74°, 23,42°, 24,32°, 24,72°, 26,05°, 27,48°, 28,10°, 29,14°, 29,79°, 30,12°, 30,61°, 31,30°, 32,06°, 32,43°, 33,36°, 34,02° et 34,38°.
